# EUROPEAN PATENT SPECIFICATION

(11) **EP 1 833 517 B1**
(45) Date of publication and mention of the grant of the patent: **24.09.2008**
(21) Application number: 05818707.1
(22) Date of filing: 12.12.2005
(51) Int. Cl.: A61L 15/32

(54) **PROCESS FOR THE PREPARATION OF TWO AND THREE DIMENSIONAL POLYMER SCAFFOLDS**
VERFAHREN ZUR HERSTELLUNG VON ZWEI- UND DREIDIMENSIONALEN POLYMERGERÜSTEN
PROCEDE DE PREPARATION D'ECHAFAUDAGES POLYMERIQUES A DEUX ET A TROIS DIMENSIONS

(30) Priority: 14.12.2004 US 635603 P
(43) Date of publication of application: 19.09.2007
(73) Proprietor: FIDIA ADVANCED BIOPOLYMERS S.R.L., 35031 Abano Terme (Padova) (IT)
(72) Inventor: PASTORELLO, Andrea, I-35031 Abano Terme (IT); PAVESIO, Alessandra, I-31141 Padova (IT)
(74) Representative: Minoja, Fabrizio
(86) International application number: PCT/EP2005/013288
(87) International publication number: WO 2006/063758

(56) References cited:
- EP-A- 0 372 966
- EP-A- 0 437 095
- WO-A-99/04828
- US-A- 2 517 772
- US-A- 5 484 913
- US-B1- 6 649 162

## Description

### SUBJECT OF THE INVENTION

The present invention describes a new process for the preparation of two- and three-dimensional polymer scaffolds on which proteins with enzymatic activity are fixed, especially coagulation factors. The scaffolds can be used in situations calling for rapid and effective haemostasis, such as during surgery, or in the case of deep wounds or trauma to the internal organs.

### BACKGROUND OF THE INVENTION

Staunching bleeding during surgery or from damaged organs or haemorrhage-prone wounds has been attempted in various ways over the years, and a partial solution has been found in the so-called 'fibrin glues' and/or tampons made of various materials enriched with factors able to activate rapid coagulation localised at the application site. Fibrin glues (such as Tisseel VH^{®}, Baxter) are usually made up extemporaneously by mixing suitable quantities of fibrinogen and thrombin which, as is known, give rise to a fibrin clot that stems the flow of blood. However, these products are not suitable for stopping heavy bleeding from an extensive area. In such cases it is preferable to use two- or three-dimensional structures enriched by various techniques (adsorption, imbibition, etc.) with factors that activate the coagulation process once the support has been fitted into the wound. The factors used are substantially fibrinogen and thrombin which, once applied, combine to form fibrin or thrombin alone, which reacts with the fibrinogen physiologically present in the lesion. The materials used to make the scaffold must be characterised by biocompatibility and bio-adhesiveness, they must be easy to process and handle and must fit into the lesion in question. Natural polymers are particularly suitable for the purpose (for instance, glycosaminoglycans, polysaccharides, cellulose, pectic acid, alginic acid, collagen, gelatine), as are semisynthetic polymers (such as derivatives of cellulose, alginic acid, hyaluronic acid, collagen cross-linked with dicarboxylic acids) or synthetic ones (including polylactic acid, polyglycolic acid and their copolymers, polycaprolactone); said polymers can also be chemically modified. For example, hyaluronic acid has been modified in various ways, such as:
- esterification (HYAFF®) with alcohols of the aliphatic, araliphatic, aromatic, cyclic and heterocyclic series (EP 216453 B1);
- amidation (HYADD^{™}) with amines of the aliphatic, araliphatic, cycloaliphatic, aromatic, cyclic and heterocyclic series (EP 1095064 B1);
- deacetylation on the fraction of N-acetyl-glucosamine (EP 1312772 B1);
- O-sulfation (EP 702699 B1);
- percarboxylation (HYOXX^{™}) by oxidation of the primary hydroxyl of the N-acetyl-glucosamine fraction (patent application EP 1339753).

Hyaluronic acid used according to the present invention may be obtained from any source for instance by extraction of cock's combs (EP 138572), by fermentation (EP 716688), or by biotechnological route. The molecular weight may range from 400 to 3x10⁶ Da, particularly from 1x 10⁵ Da to 1x 10⁶ Da, even more particularly from 200.000 to 750.000 Da.

The polymers can be made into various shapes and sizes, as described for example in EP 618817 for hyaluronic acid derivatives. A physical-type haemostatic activity has been attributed to these polymers, due solely to their excellent absorbent properties (for example, the haemostatic properties of hyaluronic acid derivatives are claimed in EP 999859) and this activity is highly desirable in the situations contemplated by the present invention. Indeed, it is already known that it is possible to exploit the presence of both fibrinogen and thrombin in a biomaterial constituted by a hyaluronic acid derivative (US 6,503,527) to obtain a tampon with haemostatic activity. One would suppose that the haemostatic activity mentioned so far is due to the activity of thrombin remaining unaltered, as it represents the key enzyme in the last stage of the coagulation cascade. Indeed, fibrinogen is not indispensable, as it is abundantly available at the lesion site. The scientific literature reports that thrombin is at its most active at pH=7, that is, the pH of blood, and this value must be kept as stable and constant as possible when thrombin is associated with a polymer, whether on its own or with fibrinogen. Any major variations in acidity may lead to a reduction in, or even the complete loss of, enzymatic activity due to the denaturation of the protein structure. The haemostatic dressings currently being developed are all characterised by a polymer scaffold of a substantially acid nature. For example, the scaffold described in US 6,503,527 is constituted by the benzyl ester of hyaluronic acid (HYAFF^{®}11) wherein 75% of the carboxy functions are esterified, while the other 25% remain free and therefore capable of interfering with the action of thrombin. Moreover, a product is already available on the market that is based on thrombin and fibrinogen on a compact and bioadhesive collagen patch (TachoSil^{®} Nycomed): in this case, the level of acidity of the product is due to the chemical processes used in transforming the starting polymer into the compact structure on which the coagulation factors are deposited. This represents a serious limitation to the products, because the activity of the enzyme, albeit present, is decidedly inferior to that which can be obtained in a situation where the pH of the scaffold, when it is applied to the lesions, is as similar as possible to that of blood, that is, pH=7. The present invention overcomes these limitations by a new process for the preparation of the polymer scaffolds that are to be imbibed with thrombin so that they can exercise their haemostatic effect on extensive surfaces and/or deep wounds, lesions to the internal organs, surgical wounds with blood loss from extensive areas. This process enables the enzymatic activity of thrombin to be completely maintained by maintaining at neutral values the pH of the two- or three-dimensional structure that acts as a scaffold for the enzyme.

### DETAILED DESCRIPTION OF THE INVENTION

The present invention concerns a new process for the neutralisation of a two- or three-dimensional polymer scaffold, so that once it has been enriched with one or more pharmacologically and/or biologically active protein interfering with the coagulation process, particularly coagulation enzymes, it can be used to induce haemostasis in cases of superficial and/or deep wounds, surgical wounds or lesions to internal organs, enabling the expression of maximum enzymatic activity.

Materials that are suitable for the purposes of the present invention can also be obtained from said polymers that have been chemically modified (for example, hyaluronic acid chemically modified by esterification, amidation, O-sulfation, deacetylation, percarboxylation), possibly associated with one another and made into various forms and sizes (films, sponges, meshes, non-woven and woven fabrics, membranes and granules).

As said before, the polymer scaffolds to which the present invention can be applied are characterised by varying degrees of acidity, attributable to the intrinsic nature of the polymer and/or its derivative, or to the methods used in processing the polymer. Acidity has a negative effect on haemostatic activity, especially that of thrombin, which ceases to have a coagulating effect when applied to the lesion. Neutralising acidity is one way of solving the problem and overcoming the limitations of the methods known to date, including when the scaffolds described herein are used to immobilise other molecules with biological-pharmacological activity (for example, growth factors such as FGF, EGF, IGF, TNF, PDGF, VEGF, BMP) that are sensitive to an acid environment.

The polymer scaffolds according to the invention, according to an alternative embodiment thereof, may accordingly be used not only for haemostatic use but also to promote wound healing, e.g. by immobilising on said polymer scaffolds a growth factor such as those mentioned above.

The process of the invention involves two steps:
- neutralisation of the scaffold with a basic substance (e.g., sodium bicarbonate) or with a buffer solution;
- drying the scaffold;
- imbibition of the scaffold with a precisely calculated amount of thrombin.

According to the type of polymer being used, it may prove necessary to pre-treat the scaffold with a solution of sodium chloride to improve absorption of the basic solution, thus increasing the ionic mobility of the medium and favouring the action of the neutralising agent. It would then be necessary to dry or freeze-dry the scaffold. The neutralising step is performed by treating the product with a basic substance (e.g. NaHCO₃) which, according to the type of material being used, is added in stoichiometric quantities or calculated excess. When stoichiometric quantities are added, the solvent used as vehicle for the basic substance must also be exactly calculated, so that the polymer maintains its ability to absorb the thrombin solution that is added immediately afterwards. Indeed, thrombin is normally supplied in a freeze-dried form or as a frozen solution, and in any case it needs to be dissolved in a solution before being spread on the product. In the case of low-acidity polymers, the thrombin can be dissolved in a buffer solution (such as PBS) which will be sufficient to combat the acidity of the scaffold.

When neutralisation is achieved by adding a calculated excess of basic substance, imbibition with NaCl and neutralisation with basic solution (e.g., NaHCO₃) should be performed at the same time, dissolving the salts in a mixture of ethanol and water in a ratio of 60:40. This operation is followed by careful rinsing to remove any excess of the base. The rinsing solutions are composed of ethanol and water, preferably in a ratio of 80:20. Finally, it is washed only with ethanol, exposed to a few passages in acetone and then dried. At this point, the scaffold is imbibed with the solution containing the biologically-pharmacologically active molecule of choice (thrombin and/or other coagulation factors, growth factors, etc).

Once they have undergone this treatment, the scaffolds are packaged, and this step must guarantee their stability and sterility. Sterility is of paramount importance in view of the application field for which the scaffolds of the present invention are intended. Sterilisation is achieved by the classic methods (including γ rays, β rays, ethylene oxide) and at different stages of the process of the invention, according to the type of polymer and pharmacologically/biologically active molecule used. More precisely, sterilisation can be performed before the scaffold is neutralised, or after neutralisation and before imbibition with the pharmacologically/biologically active substance. Indeed, the active molecule may be sensitive to the sterilisation process, and in this way it is possible to prevent it from being degraded. It must be borne in mind that after sterilisation, any other procedure must be performed in an aseptic environment; keeping all necessarily aseptic steps to a minimum diminishes the risk of contamination and makes the process of the present invention more economical on an industrial scale.

Polymer and active molecule permitting, the product can be sterilised at the very end of the process.

The present invention refers to the neutralisation process of a two- or three-dimensional scaffold based on hyaluronic acid benzyl ester, in which the carboxy functions have been esterified with benzyl alcohol at percentages varying between 75 and 100% and preferably 80% (HYAFF^{®} 11-p75HE). In all the examples, the neutralised supports received a deposit of thrombin, obtained by known methods. However, the process described herein is also applicable to the imbibition of said polymer scaffolds with other pharmacologically and/or biologically active molecules, besides enzymes in general that are sensitive to variations in acidity levels, for which it is essential to stabilise the pH of the scaffold on which they are fixed around values of between 6.5 and 7.5.

The invention is further illustrated by the following examples of neutralisation of polymer scaffolds and their subsequent imbibition with thrombin, scaffolds that are intended for use as haemostatic tampons for large wounds, damaged organs and/or surgical wounds characterised by extensive areas of bleeding.

### 1. Neutralisation of a HYAFF^{®}11 p75HE scaffold with stoichiometric quantities of NaHCO₃

### 1.1 Pre-treatment with a sodium chloride solution

A strip of non-woven HYAFF^{®}11 p75HE measuring 4 x 4 cm and weighing 152 mg is placed in a Petri dish, wetted with 1.4 ml of saline solution (0,9% of NaCl in water) and left for at least 10 minutes. It is then checked to ensure that there are no dry areas. If dry patches are still present, a few more drops of saline are added, leaving for a further 10 minutes.

The product is dried in a vacuum freeze-dryer: it is frozen to at least -30°C and then the drying chamber is depressurised to 10 (-1) millibar.

The dishes are warmed to -5°C and left for 4 hours, and then to 25°C and left for at least 6 hours.

### 1.2 Titration of the scaffold

The scaffold of non-woven HYAFF^{®}11 p75HE pre-treated with NaCl is cut up and 165 mg is weighed and placed in a beaker with 60 ml of water; 0.1 g of NaCl is added and it is shaken for another 5 minutes.

It is cooled to a constant temperature of between 1 and 5°C; 5 drops of universal indicator liquid are added and a 0.01 NaOH solution is added drop by drop until neutrality is reached, as shown by the green colouring that appears on the non-woven fabric specimen.

The amount of 0.01N NaOH consumed is 1.7 ml, corresponding to 0.017 mmol; the quantity of bicarbonate consumed is equal to MW sodium bicarbonate = 84.04 x 0.017 = 1.428 mg

### 1.3 Neutralisation of the scaffold with sodium bicarbonate

A specimen of non-woven HYAFF^{®}11 p75HE is used, prepared according to point 1.1 and sterilised by γ ray. It is necessary to prepare a 1% sodium bicarbonate solution by dissolving 1.001 g of sodium bicarbonate in 100 ml of water. 1 ml of 1% sodium bicarbonate solution is then taken and the volume is adjusted to 5.6 ml with water.
0,8 ml of this solution is then used to dampen the test sample.

### 1.4 Imbibition of the scaffold with thrombin solution

0,5 ml of a solution containing 2000 units of thrombin/ml is spread over the sample prepared according to point 1.3 and then left for at least 15 minutes.

The product is then freeze-dried as follows: the product is cooled to a temperature of between -2 and 5°C, then frozen at a temperature of less than - 30°C. the drying chamber is then depressurised to 10 (-1) millibar. The dishes are warmed to a temperature of between -25°C and -10°C then left to sublime for at least 12 hours. The dryer dishes are brought to a temperature of between -10°C and +25°C for at least 2 hours.

### 1.5 Preparation of the non-neutralised sample

A strip of non-woven HYAFF^{®}11 p75HE pre-treated with a solution of NaCl is treated with 0.8 ml of water alone and 15 minutes later 0.5 ml of thrombin solution is spread over the surface.

### 1.6 Extraction of thrombin from the samples and subsequent dilution to 5 UT/ml (Unit of Thrombin/ml)

The two different samples are inserted into a 5-ml syringe (without its plunger), and then the plunger is replaced. To each syringe is added 1.3 ml of water by means of the needle cap. This is placed to one side for about an hour.

The solution is extracted from the non-woven fabric by pushing the plunger into the syringe; the extract is collected in a test tube. The non-woven fabric is then washed by adding 1.5 ml of PBS to the syringe, leaving it for a few minutes and then extracting the solution by pressing on the plunger. The solution thus obtained is added to the test tube containing the first extract. The operation is repeated.

The solution is brought to a volume of 10 ml, obtaining a concentration of 100 UT/ml.

1 ml of the solution at a concentration of 100 UT/ml is taken and brought to a volume of 10 ml with PBS obtaining a concentration of 10 UT/ml.

1 ml of the solution at a concentration of 10 UT/ml is taken and brought to a volume of 2 ml with PBS obtaining a concentration of 5 UT/ml.

### 1.7 Preparation of a reference solution of thrombin with a concentration of 5 UT/ml

0.5 ml of thrombin solution at a concentration of 2000 UT/ml is diluted with PBS (Dulbecco's phosphate buffered saline) to 10 ml, obtaining a concentration of 100 U.T./ml.

1 ml of the solution at a concentration of 100 UT/ml is brought to a volume of 10 ml with PBS, giving a concentration of 10 UT/ml.

1 ml of the solution at a concentration of 10 UT/ml is taken and brought to a volume of 2 ml with PBS, giving a concentration of 5 UT/ml.

### 1.8 Preparation of a fibrinogen solution with a concentration of 1 mg/ml

1 ml of fibrinogen solution with a concentration of 90 mg/ml is diluted with PBS (Dulbecco's phosphate buffered saline) to a volume of 9 ml, giving a solution of fibrinogen with a concentration of 10 mg/ml. 1 ml is taken and brought to a volume of 10 ml with PBS, giving a concentration of 1 mg/ml of fibrinogen.

### 1.9 Comparison between the activity of thrombin extracted from neutralised and non-neutralised non-woven fabric

A Petri dish measuring 10 x 10 cm is placed flat on a surface without its lid. Three dabs of 100 microlitres, one from each of the three thrombin solutions prepared earlier, are placed on the dish about 1 cm from the edge, in a row about 2 cm apart:
1. reference solution with a concentration of 5 UT/ml;
2. solution extracted from the non-woven material neutralised with bicarbonate with a concentration of 5 UT/ml;
3. solution extracted from the non-neutralised non-woven material with a concentration of 5 UT/ml.

At the same time, three samples of 100 microlitres of fibrinogen with a concentration of 1 mg/ml are added (using a multi-channel pipette). This is then left to stand for between 30 and 60 seconds.
The dish is then tipped slowly until it is in a vertical position and the following is verified:
- that the reference sample constituted by fibrinogen + thrombin and the one constituted by fibrinogen + thrombin extracted from the non-woven material neutralised with bicarbonate form a clot of the same volume as the original sample and remain firmly adhered to the dish when tilted;
- that the sample formed by fibrinogen + thrombin extracted from the non-woven material that had not been neutralised with bicarbonate forms a clot that is far smaller than the other two and slides down the dish when tilted.

This indicates that in sample 3, the thrombin was unable to exercise fully its enzymatic activity because of the acidity of the scaffold.

### Reference Example

### Neutralisation of a scaffold with oxidised regenerated cellulose (ORC)

Neutralisation is achieved on this type of polymer scaffold by adding an excess of basic substance such as sodium bicarbonate.

### 2.1 Preparation of a hydroalcoholic solution of sodium bicarbonate.

0.5 grams of sodium bicarbonate is dissolved in 100 ml of water. The solution is stirred slowly while 400 ml of ethanol is added.

### 2.2 Neutralisation of the sample

A strip of tissue based on ORC (4x4 cm, weight 148 mg) is placed in the resulting mixture for at least 20 minutes. The sample is then removed from the solution and washed at least twice with 200 ml of a solution of ethanol and water in a ratio of 80:20.

The pH of the surface is tested with an indicator liquid: if it exceeds 7, further washes are performed until the value is corrected.

At this point, the sample is washed twice in 200 ml of absolute ethanol. Each wash lasts at least 15 minutes.

The sample is placed in a dryer set at 30°C in a flow of nitrogen for at least 4 hours, in a vacuum for at least 8 hours, and then sterilised by γ ray.

1.2 ml of thrombin solution (with a concentration of 840 unit/ml) is placed on the tissue.

It is left to stand for at least 20 minutes and then freeze-dried as described in point 1.1.

### 2.3 Preparation of the non-neutralised sample

A strip of ORC (cm 4x4, peso 148 mg) is immersed in a hydroalcoholic solution constituted by 100 ml of water and 400 ml of ethanol. The procedure described in point 2.2 is then followed, without checking the pH of the surface, but performing the same steps of rinsing, drying and imbibition with thrombin.

### 2.4 Comparing the activity of thrombin extracted from neutralised and non-neutralised ORC

The activity was tested as described in point 1.9 and it was shown that the thrombin maintained maximum activity; indeed, after treatment with fibrinogen solution, the sample of thrombin extracted from ORC neutralised with sodium bicarbonate and the one constituted by reference thrombin form two clots of the same size, that are far larger than the one obtained with thrombin extracted from the non-neutralised sample. Moreover, these two clots remain stuck to the Petri dish when it is tilted.

### 3. Neutralisation of a scaffold of HYAFF^{®}11 p75HE with an excess of NaHCO3

In this case, when an excess of basic substance is used, it is not necessary to weigh the non-woven HYAFF^{®}11 p75HE with any precision.

### 3.1 Preparation of the neutralising bath and neutralisation

The neutralising bath also contains an agent favouring ionic mobility, and is constituted by a mixture of ethanol and water in a ratio of 60:40.
300 ml of ethanol and 200 ml of water are then mixed together, and to this mixture is added 1.9 g of NaHCO₃ and 2.25 g of NaCl. The solution is then cooled to a temperature of 20° +/- 5° C and in it a strip of non-woven HYAFF^{®}11 p75HE weighing 10 g is immersed, suspended by two stainless steel meshes. The non-woven material is kept in the neutralising bath for about 60 minutes.

### 3.2 Washes

At least 3 washes with a mixture of ethanol and water in a ratio of 80:20 at a temperature of 20° +/- 5 C are necessary to eliminate the excess of base The strip of non-woven HYAFF^{®}11 p75HE is therefore washed by being shaken in 400 ml of the ethanol-water mixture described. The first two washes last between 1 and 2 hours each, and the subsequent ones last between 1 and 3 hours each. After the third rinse, neutralisation of the washing mixture is verified; if neutrality has not yet been achieved, further washes are performed until it has. A final wash is performed by shaking the sample in 400 ml of ethanol for at least 90 minutes.

The strip of non-woven HYAFF^{®}11 p75HE is then washed twice by shaking it in 300 ml of acetone, for at least 4 hours each time. The scaffold thus neutralised is dried in a flow of nitrogen at a temperature of 30 ° C and sterilised by γ ray.

### 3.3 Imbibition of the scaffold with thrombin solution

A solution is prepared composed of thrombin (1000 unit/ml) in PBS. About 250 mg of the sample of neutralised, sterile, non-woven HYAFF^{®}11 p75HE is placed in a syringe without its plunger. The plunger is then replaced and 1.2 ml of thrombin solution is added using the needle cap. This is left to stand for 3 hours.

### 3.4 Extraction of the thrombin solution from the sample

The liquid is squirted out of the syringe into a test tube. Another 1.5 ml of PBS is placed in the syringe and some minutes later the liquid is removed again and added to the previous quantity. This operation is repeated at least twice more, then the volume is adjusted to 10 ml with PBS.

### 3.5 Preparation of the non-neutralised sample

300 ml of ethanol is mixed with 200 ml of water, cooled to a temperature of 20° C, and in this mixture a strip of non-woven HYAFF^{®}11 p75HE weighing 10 g is immersed, suspended by two stainless steel meshes. After about 60 minutes, the cycles of rinsing, drying, sterilisation, imbibition and extraction of thrombin can be performed as described in the previous points.

### 3.6 Reference solutions

The thrombin and fibrinogen solutions are prepared as described in points 1.7 and 1.8.

### 3.7 Comparison between the activities of thrombin extracted from neutralised and non-neutralised HYAFF^{®}11 p75HE

The activities are tested as described in point 1.9.

After the addition of fibrinogen, two clots form in the samples constituted by reference thrombin and thrombin extracted from the neutralised sample. The clots are the same size and adhere to the bottom of the Petri dish when tilted. In the sample of thrombin extracted from the non-neutralised control sample, after the addition of fibrinogen a very small clot forms which rapidly slides down the wall of the Petri dish when tilted.

In this case too, therefore, neutralising the scaffold does not affect the activity of the thrombin.

The above description confirms the importance of neutralising the polymer scaffolds to be used, after imbibition with thrombin, as haemostatic agents in cases requiring the rapid coagulation of deep and/or superficial wounds, damaged organs and/or during surgery; the process claimed by the present invention overcomes the limitations of present knowledge of the problem, and provide a solution that is easy to apply, simple to make, effective and safe. It has also proved successful when the pharmacologically/biologically active molecules are other coagulation factors or growth factors.

## Claims

1. A process for the preparation of **two- and tri-dimensional** polymer scaffolds for haemostatic use containing a pharmacologically and/or biologically active protein interfering with the coagulation process, the scaffold consisting of hyaluronic acid modified by esterification, deacetylation, O-sulfation, amidation, percarboxylation, in the form of films, sponges, meshes, non-woven and woven fabrics, membranes and granules said process comprising the following steps:
• neutralisation of the **two- or tri-dimensional** scaffold with a basic substance or a buffer solution;
• drying the scaffold;
• imbibition of the scaffold with a predefined quantity of said pharmacologically and/or biologically active protein.

2. The process according to any one of claims 1 wherein hyaluronic acid is esterified with benzyl alcohols to a degree of between 75 and 100%.

3. The process according to claim 1 wherein neutralisation is performed with alkaline bicarbonates or buffer solutions.

4. A process according to claims 1-3 wherein sterilisation is performed before neutralisation of the scaffold.

5. A process according to claims 1-3 wherein sterilisation is performed after imbibition of the scaffold with the biologically/pharmacologically active substance.

6. A process according to claims 1-3 wherein sterilisation is performed after neutralisation of the scaffold and before imbibition with the biologically/pharmacologically active substance.

7. The process according to claim 1 wherein the biologically/pharmacologically active protein is a coagulation factor, particularly thrombin.

8. The process according to claim 1 wherein the biologically/pharmacologically active molecule is a growth factor.

9. The process according to claim 8 wherein the molecule is selected from FGF, EGF, IGF, TNF, PDGF, VEGF, BMP.

10. Polymer scaffolds obtainable by any one of the process of claims 1-7.

11. The use of polymer scaffolds of claim 10 for the preparation of devices for haemostatic use.

12. Polymer scaffolds obtainable by the process of claims 8 or 9.

13. The use of polymer scaffolds of claim 12 for the preparation of devices for promoting wound healing.

## Patentansprüche

1. Verfahren zur Herstellung eines zwei- und dreidimensionalen Polymergerüsts zur blutstillenden Verwendung, das ein pharmakologisch und/oder biologisch aktives Protein enthält, das in den Gerinnungsvorgang eingreift, wobei das Gerüst aus durch Veresterung, Deacetylierung, O-Sulfatierung, Amidierung, Percarboxylierung modifizierter Hyaluronsäure in der Form von Folien, Schwämmen, Netzen, Vliesen und Geweben, Membranen und Granula besteht, wobei das Verfahren die nachstehenden Schritte umfasst:
• Neutralisation des zwei- oder dreidimensionalen Gerüsts mit einer basischen Substanz oder einer Pufferlösung;
• Trocknen des Gerüsts;
• Imbibition des Gerüsts mit einer vordefinierten Menge des pharmakologisch und/oder biologisch aktiven Proteins.

2. Verfahren gemäß Anspruch 1, wobei die Hyaluronsäure bis zu einem Grad von 75 bis 100% mit Benzylalkoholen verestert ist.

3. Verfahren gemäß Anspruch 1, wobei die Neutralisation mit alkalischen Bicarbonaten oder Pufferlösungen erfolgt.

4. Verfahren gemäß Ansprüchen 1-3, wobei vor der Neutralisation des Gerüsts eine Sterilisation erfolgt.

5. Verfahren gemäß Ansprüchen 1-3, wobei die Sterilisation nach der Imbibition des Gerüsts mit der biologisch/pharmakologisch aktiven Substanz erfolgt.

6. Verfahren gemäß Ansprüchen 1-3, wobei die Sterilisation nach der Neutralisation des Gerüsts und vor der Imbibition mit der biologisch/pharmakologisch aktiven Substanz erfolgt.

7. Verfahren gemäß Anspruch 1, wobei das biologisch/pharmakologisch aktive Protein ein Gerinnungsfaktor, insbesondere Thrombin, ist.

8. Verfahren gemäß Anspruch 1, wobei das biologisch/pharmakologisch aktive Molekül ein Wachstumsfaktor ist.

9. Verfahren gemäß Anspruch 8, wobei das Molekül ausgewählt ist aus FGF, EGF, IGF, TNF, PDGF, VEGF, BMP.

10. Polymergerüst, das durch ein jegliches Verfahren der Ansprüche 1-7 erhältlich ist.

11. Verwendung des Polymergerüsts nach Anspruch 10 zur Herstellung von Vorrichtungen zur blutstillenden Verwendung.

12. Polymergerüst, das durch das Verfahren nach Ansprüchen 8 oder 9 erhältlich ist.

13. Verwendung des Polymergerüsts nach Anspruch 12 zur Herstellung von Vorrichtungen zur Förderung von Wundheilung.

## Revendications

1. Procédé de préparation de squelettes polymériques bi- ou tri-dimensionnels pour une utilisation hémostatique contenant une protéine pharmacologiquement et/ou biologiquement active interférant avec le processus de coagulation, le squelette consistant en de l'acide hyaluronique modifié par estérification, déacylation, O-sulfatation, amidation, percarboxylation, sous forme de films, d'éponges, de filets, d'étoffes tissées ou non-tissées, de membranes et de granules, ledit procédé comprenant les étapes suivantes :
■ la neutralisation du squelette bi- ou tri-dimensionnel avec une substance basique ou une solution tampon ;
■ le séchage du squelette ;
■ l'imbibition du squelette avec une quantité prédéfinie de ladite protéine pharmacologiquement et/ou biologiquement active.

2. Procédé selon la revendication 1, dans lequel l'acide hyaluronique est estérifié avec des alcools benzyliques à un degré compris entre 75 et 100 %.

3. Procédé selon la revendication 1, dans lequel la neutralisation est effectuée avec des bicarbonates alcalins ou des solutions tampons.

4. Procédé selon l'une quelconque des revendications 1-3, dans lequel la stérilisation est effectuée avant la neutralisation du squelette.

5. Procédé selon l'une quelconque des revendications 1-3, dans lequel la stérilisation est effectuée après l'imbibition du squelette avec la protéine biologiquement / pharmacologiquement active.

6. Procédé selon l'une quelconque des revendications 1-3, dans lequel la stérilisation est effectuée après la neutralisation du squelette et avant l'imbibition du squelette avec la protéine biologiquement / pharmacologiquement active.

7. Procédé selon la revendication 1, dans lequel la protéine biologiquement /pharmacologiquement active est un facteur de coagulation, en particulier la thrombine.

8. Procédé selon la revendication 1, dans lequel la protéine biologiquement /pharmacologiquement active est un facteur de croissance.

9. Procédé selon la revendication 8, dans lequel la molécule est sélectionnée parmi FGF, EGF, IGF, TNF, PDGF, VEGF et BMP.

10. Squelettes polymériques susceptibles d'être obtenus par un quelconque procédé selon les revendications 1-7.

11. Utilisation de squelettes polymériques selon la revendication 10 pour la préparation de dispositifs destinés à une utilisation hémostatique.

12. Squelettes polymériques susceptibles d'être obtenus par le procédé selon les revendications 8 ou 9.

13. Utilisation de squelettes polymériques selon la revendication 12 pour la préparation de dispositifs destinés à promouvoir la cicatrisation d'une plaie.
